# EUROPEAN PATENT SPECIFICATION

(11) **EP 0 247 881 B1**
(45) Date of publication and mention of the grant of the patent: **13.01.1993**
(21) Application number: 87304745.0
(22) Date of filing: 28.05.1987
(51) Int. Cl.: C12N 5/00

(54) **Process for culturing animal cells and appropriate media**
Verfahren zur Züchtung tierischer Zellen und geeignete Medien
Procédé pour la culture de cellules animales et milieux appropriés

(30) Priority: 28.05.1986 JP 121239/86
(43) Date of publication of application: 02.12.1987
(73) Proprietor: TOA NENRYO KOGYO KABUSHIKI KAISHA, Tokyo 100 (JP)
(72) Inventor: Shinohara, Kazuka, Niihari-gun, Ibaraki (JP); Koyano, Takashi, Nakano-ku Tokyo (JP)
(74) Representative: Bizley, Richard Edward

(56) References cited:
- EP-A- 0 049 632
- EP-A- 0 067 697
- CHEMICAL ABSTRACTS, vol. 92, 1980, page 471, abstract no. 213534p, Columbus, Ohio, US; & JP-A-80 18 449 (DAINIPPON INK AND CHEMICALS, INC.) 08-02-1980
- CHEMICAL ABSTRACTS, vol. 91, 1979, page 542, abstract no. 209680v, Columbus, Ohio, US; & JP-A-79 95 770 (DAINIPPON INK AND CHEMICALS, INC.) 28-07-1979

## Description

The present invention relates to a process for culturing animal cells.

Recently, various kinds of physiologically active compounds such as interferons, fibrinolitic enzyme activators, and lymphokines, which are originally produced in vivo and exhibit their action in situ, can be produced by culturing animal cells such as hybridoma cells in vitro and recovering the product from a supernatant of the culture, and such products are promising pharmaceuticals. To accomplish this purpose, however, the producer animal cells must be stably grown in a medium.

In many cases, a medium containing a serum is necessary to culture animal cells. However, since serum contains various kinds of substances such as vitamins hormones, amino acids, and proteins, and the contents of such substances vary lot by lot, it is difficult to obtain serum preparations having consistent properties, resulting in inconsistent culture results. Moreover, when the components contained in a serum have similar properties to those of a target product, isolation of the purified product from the medium is difficult. Also, since it is difficult to obtain a large amount of serum at a low cost, commercial production of the useful target substance in a serum-containing medium is difficult from the economical point of view.

Accordingly, serum-free methods for culturing animal cells, in particular the replacement of serum as an additive for a medium for culturing animal cells, are sought. Recently, growth factors for animal cells have been under extensive research, and for such purposes, for example, insulin, transferrin, ethanolamine are known.

The present inventors found that an extract of Synechococcus cells is useful as a replacement for serum (Japanese patent Application No. 59-41997). However, compounds derived from plants are not yet known as a replacement for serum.

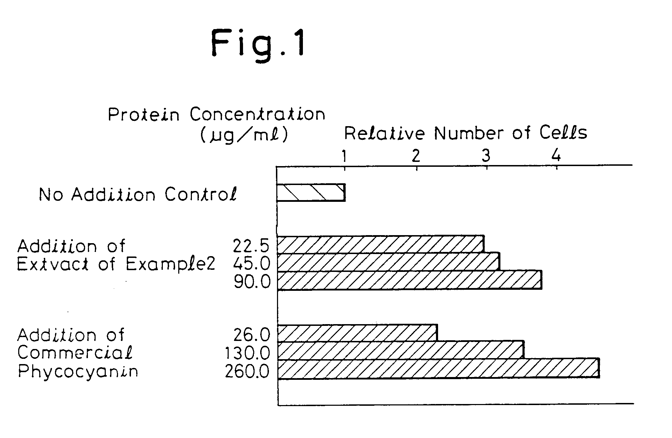

The present invention provides a process for culturing animal cells, comprising the steps of:
inoculating a medium comprising a basal medium and 20 to 300 µg/ml of phycocyanin with target animal cells; and
incubating the medium to allow the growth of the target animal cells.
In the drawing:-
Figure 1 is a graph for comparing a control medium containing no phycocyanin and media containing phycocyanin at different concentrations, for a protein concentration representing an amount of cell growth.

According to the present invention, as a medium for culturing animal cells, a basal medium supplemented with phycocyanin is used. As the basal medium, any conventional medium for culturing animal cells can be used. Such basal media include, for example, PRM1 1640, Dulbecco's Modified Eagle Medium (DME), Ham-F12, 199, Eagle's MEM and BGJ6, and a mixture thereof. These basal media are well known in the art. These basal media can be supplemented with a small amount of serum.

Phycocyanin is a blue chromoprotein found in cells of algae, i.e., Cyanophyta, Rodophyta and Cryptophyta, and is well known in the art. In the present invention, any kind of phycocyanin prepared from the above-mentioned algae can be used. The preparation process and purity of the phycocyanin are not critical for the present purpose. For example, an algae producing phycocyanin is cultured according to the conventional method, and the cultured algae cells are separated and the cells disrupted by sonication. The disruptant is then subjected to extraction to obtain an dialysis residue. The residue is subjected to gel filtration and ion exchange chromatography to purify the phycacyanin. Alternatively, phycocyanin preparations are commercially available. The concentration of phycocyanin in a medium depends on the particular basal medium, the particular animal cells to be cultured, the purpose of the culturing and the like, and is usually 20 to 300 µg/ml.

The present process can be applied to the culturing of various kinds of animal cells, including cells of established cell lines and of hybridoma cell lines. Not-established animal cells include, for example, leucocytes isolated from peripheral blood, lymphocytes isolated from lymphoid tissue and the like, of human or other animal origin. Established cell lines include myeloma cell lines such as human myeloma cell line RPM1 8226, T-lymphoid cell lines such as human T-lymphoid cell line Molt-4, B-lymphoid cell lines such as human-B-lymphoid cell line HO-323, and the like. Hybridoma cell lines include human-human hybridoma cell lines, human-murine hybridoma cell lines, murine-murine hybridoma cell lines, and the like.

Although culturing according to the present invention can be started with a stock culture of animal cells, preferably, prior to culturing according to the present invention, a preculture is carried out to prepare inoculum cells. The preculture can be carried out in any conventional medium, such as one of the above-mentioned basal media, preferably supplemented with about 10% serum, under the culture condition as described below for culturing the present invention. The cultured cells are collected and optionally washed in a conventional manner to prepare an inoculum.

Culturing according to the present invention is started by adding a stock culture or the above-prepared inoculum into a medium of the present invention described above. The culturing can be carried out according to a conventional method. A temperature for culturing is about 25°C to 45°C, preferably 30°C to 40°C, for example, 37°C. Culturing is preferably carried out under an atmosphere of 5% carbon dioxide.

After culturing, the cultured product is processed according to purpose of the culturing. For example, if a useful compound such as a monoclonal antibody is to be recovered from a supernatant, the supernatant is collected by a conventional procedure such as centrifugation. Alternatively, if cultured cells per se are a target product, or a target compound is to be isolated from the cultured cells, the cultured cells are collected according to a conventional procedure such as centrifugation.

According to the present process, animal cells, which cannot be cultured in a serum-free medium, can be cultured in a medium wherein all or a major portion of serum has been replaced by phycocyanin. Since such improved media do not contain components whose amounts differ lot by lot, the improved media can be prepared with a good reproducibility. Moreover, when animal cells are cultured in the improved medium of the present invention, to produce a target compound, the target compound can be easily recovered from the cultured broth and purified with a high purity.

### EXAMPLES

The present invention will now be further illustrated by, but is by no means limited to, the following examples.

### Example 1

In a medium comprising a mixture of RPMI 1640, DME (Dulbecco's Modified Eagle Medium) and Ham F-12 at the volume ratio of 2:1:1 (RDF medium) supplemented with 10% of FCS (fetal calf serum), cells of human myeloma cell line RPMI 8226 were cultured at a temperature of 37°C under the presence of 5% carbon dioxide for 4 days, and the cultured cells were washed with the RDF medium to eliminate the serum.

2 ml of a medium comprising the RDF supplemented with 26 to 260 µg/ml of commercially available phycocyanin was put in a 2 ml culture Petri dish, and the medium was inoculated with the above-mentioned washed cell at a final concentration of about 5 x 10⁴ cells/ml. The whole was then incubated at 37°C for 4 days under the presence of 5% carbon dioxide, and after the culturing, the number of cells was measured with a cell counter. As a control, cells were cultured in the RDF supplemented with no phycocyanin. The result is shown in Fig. 1 by the ratio of the number of cells in the test medium relative to the number of cells immediately after the inoculation of the cells.

As seen from Fig. 1, at a concentration of phycocyanin ranging from 26 µg/ml to 260 µg/ml, the number of cultured cells increased by about 2 to 4 times relative to the number of inoculated cells, depending on the concentration of added phycocyanin. On the other hand, in the control medium containing no phycocyanin, an increase of the cell number was not observed.

### Example 2

Cells of blue-green algae Synechococcus elongatus were cultured according to a conventional procedure, and the cultured cells were collected. The cells were disrupted by an ultrasonic treatment and the treated product was centrifuged to obtain an extract. The extract was dialyzed against a phosphate buffered saline (PBS), and the dialysis residual fraction was fractionated by gel filtration (Sepharose B4, at a room temperature), and then by ion exchange chromatography (DEAE-Sepharose CL-6B, at a room temperature) to obtain a phycocyanin fraction.

Using the phycocyanin fraction thus obtained, the same procedure as described in Example 1 was repeated. A concentration of protein derived from the added phycocyanin fraction in test medium ranged from 22.5 µg/ml to 90.0 µg/ml. As shown in Fig. 1, in this concentration range, the number of cultured cells increased about 3 to 4 times relative to the number of inoculated cells depending on the protein concentration.

### Example 3

The same procedure as described in Example 1 was repeated except that either cells of human T-lymphoid cell line Molt-4 or cells of human B-lymphoid cell line HO-323 were cultured, and substantially the same result was obtained as in Example 1.

## Claims

1. A process for culturing animal cells comprising the steps of:
inoculating a medium comprising a basal medium and 20 to 300 µg/ml of phycocyanin with target animal cells; and
incubating said medium to allow the growth of the target animal cells.

2. A process for culturing animal cells according to claim 1 wherein the basal medium is a conventional medium used to culture animal cells.

3. A process for culturing animal cells according to claim 1 wherein the medium comprises RPM1 1640, DME, Ham-F12, 199, Eagle's MEM, BGJ6, or a mixture thereof.

4. A process for culturing animal cells according to any one of claims 1 to 3 wherein the phycocyanin is derived from cells of an algae selected from the group consisting of Cyanophyta, Rodophyta and Cryptophyta.

5. The use of phycocyanin as a medium component in the culturing of animal cells.

6. An animal cell culture medium comprising conventional ingredients therefor and phycocyanin.

## Patentansprüche

1. Ein Verfahren zum Züchten von Tierzellen, das die Schritte aufweist:
Impfen eines Mediums, das ein Basalmedium und 20 bis 300 µg/ml Phykocyanin enthält, mit Ziel-Tierzellen;
und
Inkubieren des Mediums, um die Ziel-Tierzellen wachsen zu lassen.

2. Ein Verfahren zum Züchten von Tierzellen nach Anspruch 1, bei dem das Basalmedium ein übliches, zum Züchten von Tierzellen verwendetes Medium ist.

3. Ein Verfahren zum Züchten von Tierzellen nach Anspruch 1, bei dem das Medium RPM1 1640, DME, Ham-F12, 199, Eagle's MEM, BGJ6 oder eine Mischung davon, aufweist.

4. Ein Verfahren zum Züchten von Tierzellen nach einem der Ansprüche 1 bis 3, bei dem das Phykocyanin abstammt aus Zellen von Algen, die ausgewählt sind aus der Gruppe bestehend aus Cyanophyta, Rodophyta und Cryptophyta.

5. Die Verwendung von Phykocyanin als ein Mediums-Bestandteil beim Züchten von Tierzellen.

6. Ein Tierzellen-Kulturmedium, das übliche Zutaten dafür und Phykocyanin enthält.

## Revendications

1. Un procédé pour la culture de cellules animales, comprenant les étapes consistant à:
inoculer un milieu comprenant un milieu basique et 20 à 300 µg/ml de phycocyanine avec des cellules animales cibles; et
incuber le milieu pour permettre la croissance des cellules animales cibles.

2. Un procédé pour la culture de cellules animales suivant la revendication 1, dans lequel le milieu basique est un milieu conventionnel utilisé pour la culture de cellules animales.

3. Un procédé pour la culture de cellules animales suivant la revendication 1, dans lequel le milieu comprend RPMI 1640, DME, Ham-F12, 199, MEM d'Eagle, BGJ6, ou un mélange de ceux-ci.

4. Un procédé pour la culture de cellules animales suivant l'une ou l'autre des revendications 1 à 3, dans lequel la phycocyanine est dérivée de cellules d'une algue sélectionnée parmi le groupe composé de Cyanophyta, Rodophyta et Cryptophyta.

5. L'utilisation de phycocyanine comme composant du milieu dans la culture de cellules animales.

6. Un milieu de culture de cellules animales comprenant les ingrédients conventionnels pour celui-ci et de la phycocyanine.
